# EUROPEAN PATENT APPLICATION

(11) **EP 2 317 522 A1**
(43) Date of publication of application: **04.05.2011**
(21) Application number: 10189388.1
(22) Date of filing: 29.10.2010
(51) Int. Cl.: G21K 5/10, A61N 5/10

(54) **Accelerated particle irradiation equipment**

(30) Priority: 29.10.2009 JP 2009249364
(71) Applicant: Sumitomo Heavy Industries, LTD., Tokyo 141-6025 (JP)
(72) Inventor: Yajima, Satoru, Ehime 792-8588 (JP); Tachikawa, Toshiki, Ehime 792-8588 (JP)
(74) Representative: Wagner & Geyer

(57) **Abstract**

Accelerated particle irradiation equipment, which performs irradiation of accelerated particles, includes an irradiation device and a building. The irradiation device includes a rotating unit rotatable about a rotation axis and performs irradiation of the accelerated particles generated by a particle accelerator. The building has an installation space in which the irradiation device is installed. The irradiation device is formed to be thin so as to have a small length in a direction of the rotation axis. A portion of the irradiation device, which has the maximum width in a radial direction orthogonal to the direction of the rotation axis, is disposed along the maximum width of the installation space.

## Description

### Related Application

Priority is claimed to Japanese Patent Application No.2009-249364, filed October 29, 2009, the entire content of which is incorporated herein by reference.

### BACKGROUND

### Technical Field

The present invention relates to accelerated particle irradiation equipment that includes an irradiation device such as a rotating gantry for radiation therapy.

### Description of the Related Art

Equipment that performs cancer treatment by Irradiating a patient with accelerated particles such as a proton beam is knows, This kind of equipment includes a cyclotron that generates accelerated particles, a rotatable irradiation device (rotating gantry) that irradiates a patient with accelerated particles in an arbitrary direction, and a guide line that guides the accelerated particles generated by the cyclotron to the irradiation device. The rotating gantry is provided with a treatment table on which a patient lies, an irradiation unit that irradiates the patient with accelerated particles, and an introduction line that introduces the accelerated particles guided by the guide line into the irradiation unit.

The irradiation unit is freely rotatable relative to a patient, and various types of introduction line that introduce accelerated particles into the irradiation unit are known. For example, as a first aspect, there is known an introduction line that includes a connection portion that is connected to a guide line on a rotation axis serving as a rotation center of an irradiation units. The introduction line is curved in a substantially U shape on a plane passing through the rotation axis, and is connected to the irradiation unit. Further, as a second aspect, there is known an introduction line that includes a connection portion that is connected to a guide line on a rotation axis. The introduction line is curved so as to be twisted in the circumferential direction of the rotation axis, and is connected to an irradiation unit.

### SUMMARY

According to an embodiment of the invention, there is provided accelerated particle irradiation equipment that performs irradiation of accelerated particles. The accelerated particle irradiation equipment includes an irradiation device that includes a rotating unit rotatable about a rotation axis and performs irradiation of the accelerated particles generated by a particle accelerator, and a building having an installation space in which the irradiation device is installed. The irradiation device is formed to be thin so as to have a small length in a direction of the notation axis. A portion of the irradiation device, which has the maximum width in a radial direction orthogonal to the direction of the rotation axis, is disposed along the maximum width of the installation space.

### BRIEF DESCRIPTION OF THE DRAWINGS

Fig. 1 is a view showing the disposition of a particle radiation therapy equipment according to an embodiment of the invention,
Fig. 2 is a side view of the particle radiation therapy equipment according to the embodiment of the invention.
Fig. 3 is a perspective view of a rotating gantry according to an embodiment of the invention.
Fig. 4 is a schematic cross-sectional view of the rotating gantry according to the embodiment of the invention taken along a rotation axis in a horizontal direction.
Fig. 5 is an enlarged plan view of a gantry chamber according to an embodiment of the invention.
Fig. 6 is a cross-sectional view of the gantry chamber shown in Fig. 5 taken along a long-side direction X as seen from the rear side of a building.
Fig. 7 is a cross-sectional view of the gantry chamber shown in Fig. 5 taken along a vertical plane including a rotation axis as seen from the side of the rotating gantry.
Fig. 8 is a cross-sectional view of the gantry chamber shown in Fig. 5 taken along a plane orthogonal to the rotation axis as seen from the rear side of the rotating gantry.
Fig. 9 is a view illustrating a procedure for constructing a shield member that shields a cutout portion of a ceiling.

### DETAILED DESCRIPTION

However, in equipment including the rotating gantry of the first aspect, it is difficult to appropriately guide accelerated particles, and to reduce the path length of the introduction line in the direction of the rotation axis due to the need for introduction. Accordingly, it is difficult to reduce the dimensions of the rotating gantry in the direction of the rotation axis. As a result, since it is difficult to reduce the size of this kind of rotating gantry and a large installation space is needed to store the rotating gantry, the size of a facility increases and it is difficult to reduce the equipment coasts. Further, in the equipment of the second aspect, the disposition of the rotating gantry in a building is not considered and the avoidance of the increase of the size of the building and the reduction of the equipment costs are not sufficient.

It is desirable to provide accelerated particle irradiation equipment that may facilitate the reduction of the size of a building in which an irradiation device is installed and is effective in regards to the reduction of the equipment costs.

The irradiation device of the accelerated particle irradiation equipment according to an embodiment of the invention is formed to be thin so as to have a small length in the direction of the rotation axis. A portion of the thin irradiation device, which has the maximum width, is short in the direction of the rotation axis. If this portion is disposed along the maximum width of the installation space, it may be possible to effectively utilize an installation space. For example, if a portion of the irradiation device having the maximum width is disposed along a diagonal line of the installation space when the installation space is a rectangular area, the length and width of the rectangular area consequently become short. Accordingly, it may be possible to reduce the size of the building. As a result, it may be possible to reduce the construction costs of the building, so that it is effective in regards to the reduction of the equipment costs. Further, since it may be possible to effectively utilize an installation space, it may be possible to construct the particle radiation therapy equipment at a site smaller than a site in the related art.

Furthermore, the irradiation device may include an irradiation unit that irradiates an irradiation target with the accelerated particles, an introduction line that introduces the accelerated particles into the irradiation unit, and a weight unit that is provided so as to face the introduction line with the rotation axis therebetween and secures a weight balance between the weight unit and the introduction line. A distance between the rotation axis and a portion of the weight unit having the maximum outer diameter may be smaller than a distance between the rotation axis and a portion of the introduction line having the maximum outer diameter. Since there are many restrictions in the design of the path of the introduction line, it is difficult to reduce the dimensions of the introduction line in the radial direction orthogonal to the direction of the rotation axis. Accordingly, if the distance between the rotation axis and a portion of the weight unit having the maximum outer diameter is made to be smaller than the distance between the rotation axis and a portion of the introduction line having the maximum outer diameter, unnecessary protrusions are removed. As a result, it may be possible to facilitate the reduction of the size of the irradiation device.

In addition, the rotating unit of the irradiation device may include a main body of the rotating unit that includes the irradiation unit, and a circumferential introduction line that is an introduction line curved in a circumferential direction on the outside of the main body of the rotating unit in the radial direction. The width of the weight unit in the direction of the rotation axis may be smaller than that of the circumferential introduction line in the direction of the rotation axis. Since there are many restrictions in the design of the path of the introduction line, it is difficult to reduce the width of the introduction line in the direction of the rotation axis. Accordingly, if the width of the weight unit in the direction of the rotation axis is made to be smaller than the width of the circumferential introduction line in the direction of the rotation axis, it is advantageous in the reduction of the thickness of the Irradiation device. As a result, it may be possible to facilitate the reduction of the size of the irradiation device.

Moreover, the length of the irradiation device in the direction of the rotation axis may be smaller than a radius of rotation of the irradiation device corresponding to the maximum width. Accordingly, if the length of the irradiation device in the direction of the rotation axis is made to be smaller than the maximum width of the irradiation device, it is advantageous in the reduction of the thickness of the irradiation device. As a result, it may be possible to facilitate the reduction of the size of the irradiation device.

According to an embodiment of the invention, it may be possible to facilitate the reduction of the size of a building, thereby being effective in the reduction of the equipment costs.

Accelerated particle irradiation equipment according to a preferred embodiment of the invention will be described below with reference to drawings. A case where accelerated particle irradiation equipment is used as a particle radiation therapy equipment will be described in this embodiment. The particle radiation therapy equipment is applied to, for example, cancer treatment, and is an apparatus for irradiating a tumor (irradiation target), which exists in a patient's body, with a proton beam (accelerated particles).

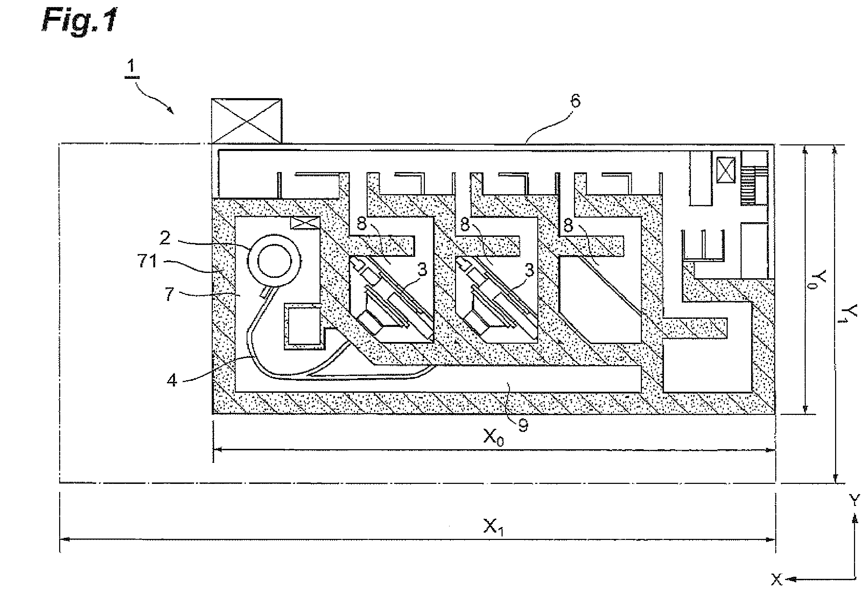

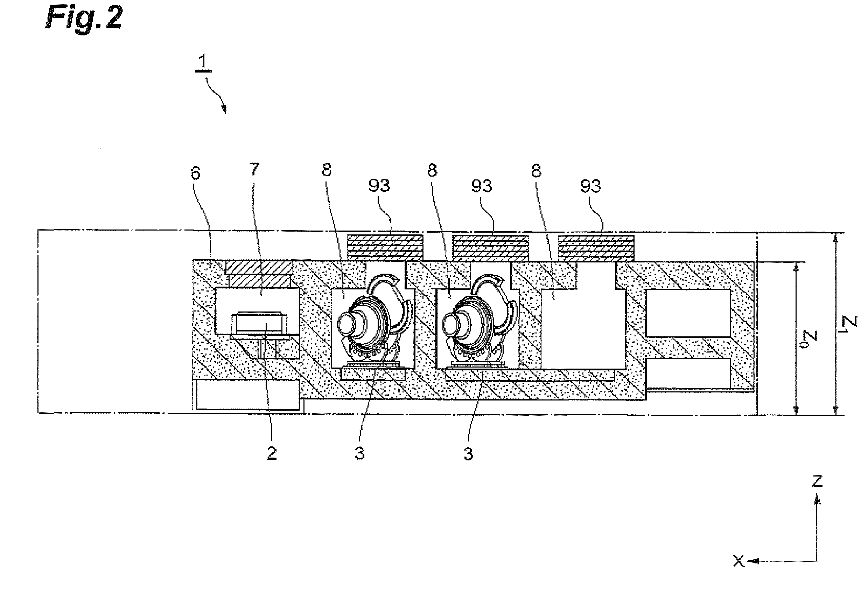

As shown in Figs. 1 and 2, the particle radiation therapy equipment 1 includes a cyclotron (particle accelerator) 2 that generates a proton beam, rotating gantries (irradiation devices) 3 that are rotatable and irradiate a patient with a proton beam in an arbitrary direction, and a guide line 4 that guides the proton beam generated by the cyclotron 2 to the rotating gantry 3. A particle radiation therapy system includes the cyclotron 2, the rotating gantries 3, and the guide line 4 as respective devices. Further, the particle radiation therapy equipment 1 includes a building 6 in which the respective devices of the particle radiation therapy system are disposed.

The particle radiation therapy system will be described. The path of a proton beam generated by the cyclotron 2 is changed along the guide line 4, and the proton beam is guided to the rotating gantries 3. The guide line 4 is provided with deflecting magnets that change the path of the proton bear.

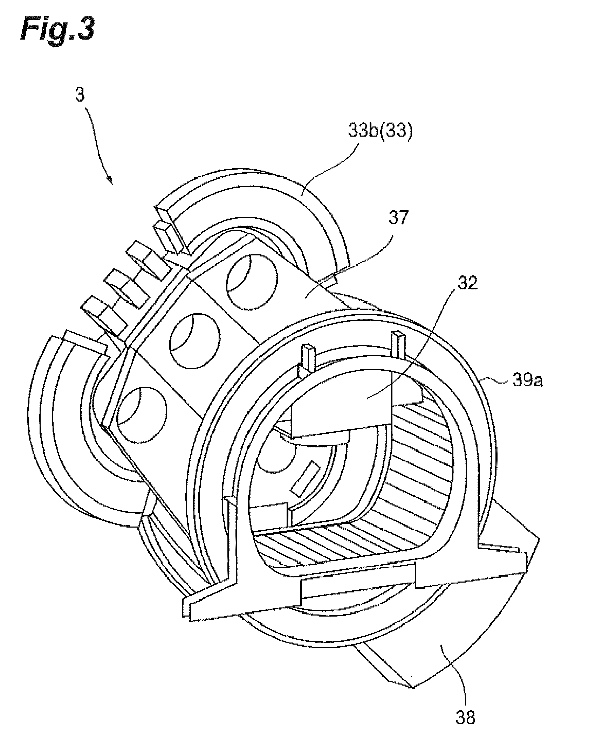

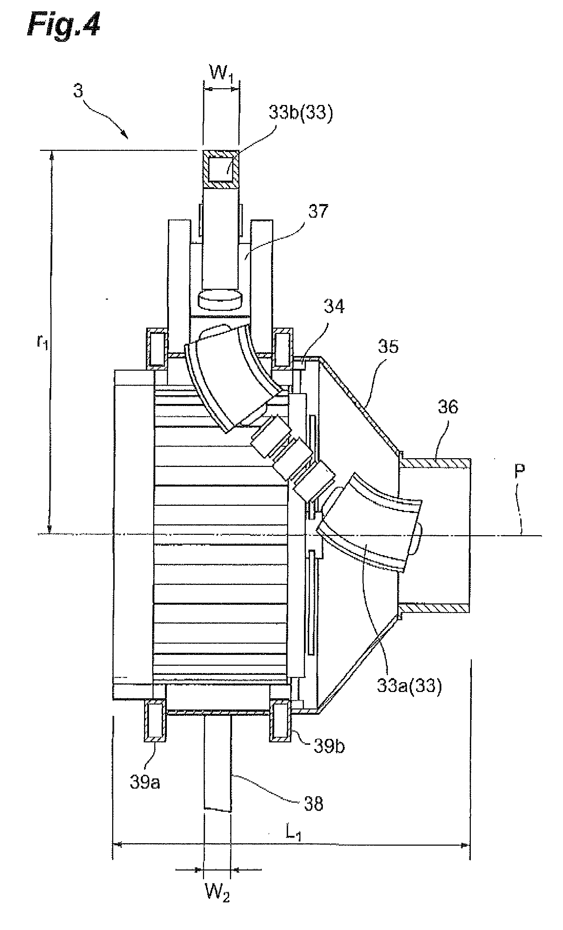

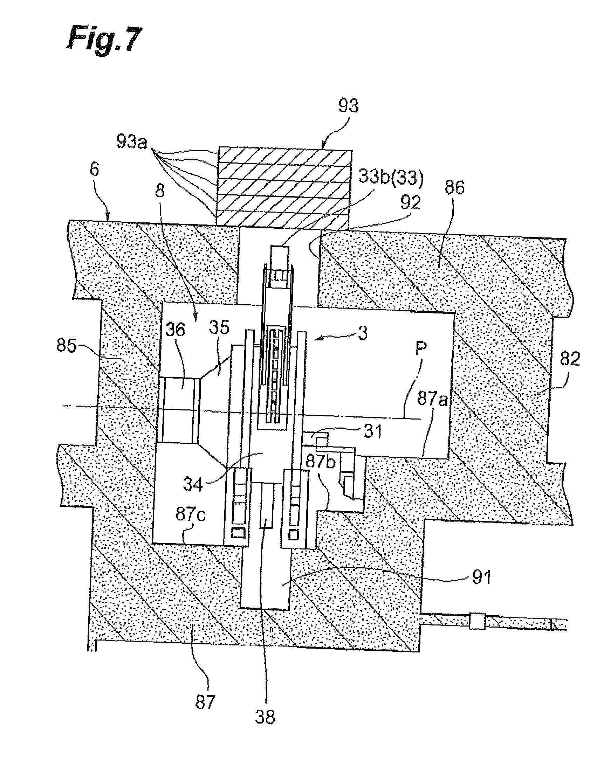

Fig. 3 is a perspective view of the rotating gantry, and Fig. 4 is a schematic cross-sectional view of the rotating gantry taken along a rotation axis in a horizontal direction. The rotating gantry 3 includes a treatment table 31 (see Fig. 7) on which a patient lies, an irradiation unit 32 that irradiates the patient with a proton beam, and an introduction line 33 that introduces the proton beam guided by the guide line 5 into the irradiation unit 32.

The rotating gantry 3 is rotatable and is provided with a first cylindrical portion 34, a cone portion 35, and a second cylindrical portion 36 in this order from the front side. The first cylindrical portion 34, the cone portion 35, and the second cylindrical portion 36 are coaxially disposed and connected to one another. The irradiation unit 32 of the rotating gantry 3 is disposed on the inner surface of the first cylindrical portion 34, and faces the axis of the first cylindrical portion 34. The treatment table 31 (not shown in Figs. 3 and 4) is disposed on the axis of the first cylindrical portion 34. The diameter of the second cylindrical portion 36 is smaller than that of the first cylindrical portion 34, and the cone portion 35 is formed in a conical shape so as to connect the first cylindrical portion 34 to the second cylindrical portion 36.

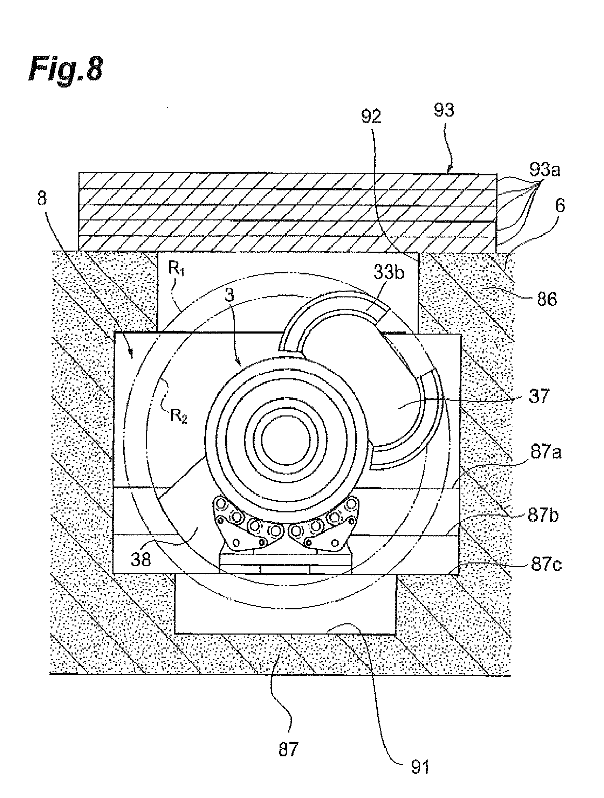

A front ring 39a is disposed at the outer peripheral portion of the front end of the first cylindrical portion 34, and a rear ring 39b is disposed at the outer peripheral portion of the rear end of the first cylindrical portion 34. As shown in Fig. 8, the first cylindrical portion 34 is rotatably supported by a roller device 40 that is disposed below the first cylindrical portion 34. The outer peripheral surfaces of the front and rear rings 39a and 39b come into contact with the roller device 40, and torque is applied to the front and rear rings by the roller device 40.

The guide line 4, which guides a proton beam to the rotating gantries 3, is connected to the rear sides of the rotating gantries 3. The guide line 4 is connected to the irradiation unit 32 by the introduction line 33. The introduction line 33 is provided with two sets of deflecting magnets corresponding to 45° and two sets of deflecting magnets corresponding to 135°, The introduction line 33 includes a radial introduction line 33a that extends in a radial direction and a circumferential introduction line 33b that is connected to the rear end of the radial introduction line 33a and extends in a circumferential direction.

After being disposed in the direction of a rotation axis P in the second cylindrical portion 36, as shown in Fig. 4 the radial introduction line 33a is bent at an angle of 90° (45° × two times) from the direction of the rotation axis P, advances to the outside in the radial direction, and protrudes to the outside of the first cylindrical portion 34 in the radial direction. As shown in Fig. 3, the circumferential introduction line 33b is bent at an angle of 135° from the radial direction, advances upward in the circumferential direction, is bent inward in the radial direction at an angle of 135°, and faces inward in the radial direction.

The circumferential introduction line 33b is disposed in a circumferential direction at a position, which is outwardly distant from the outer peripheral surface of the first cylindrical portion 34, above the outer surface of the first cylindrical portion 34. A mount 37, which supports the circumferential introduction line 33b, is provided on the outer peripheral surface of the first cylindrical portion 34. The mount 37 is formed so as to protrude outward in the radial direction, and supports the circumferential introduction line 33b.

Further, a counter weight 38 is provided on the outer peripheral surface of the first cylindrical portion 34 so as to face the introduction line and the mount with the rotation axis P interposed therebetween. The counterweight 38 is disposed so as to protrude outward from the outer peripheral surface of the first cylindrical portion 34. Since the counter weight 38 is provided, a weight balance between the counter weight and the mount 37 and the introduction line 33 disposed on the outer surface of the first cylindrical portion 34 is secured. Furthermore, it is preferable that a distance between the rotation axis P and the outer edge of the counter weight 38 be smaller than a distance between the rotation axis P and the outer edge of the introduction line 33.

Moreover, the rotating gantry 3 is rotationally driven by a motor (not shown) and the rotation of the rotating gantry is stopped by a brake device (not shown). Meanwhile, a portion, which includes the first cylindrical portion 34, the introduction line 33, and the counter weight 38, corresponds to a rotating unit of the rotating gantry 3. Further, the main body of the rotating unit is, for example, a cylindrical body of which an axis is disposed on the rotation axis, a cylindrical body that has an outer peripheral surface on the entirety of the same circumference, or a body regarded as a cylindrical body that has an outer peripheral surface on the entirety of the same circumference or the like. In this embodiment, the main body of the rotating unit corresponds to the first cylindrical portion 34.

Furthermore, the circumferential introduction line 33b and the counter weight 38 correspond to a protruding portion that protrudes further outward in the radial direction as compared to the main body of the rotating unit. In this embodiment, the introduction line 33, which is disposed on the outer surface of the first cylindrical portion 34, corresponds to the protruding portion that forms a peripheral edge portion of the rotating unit. If the distance between the rotation axis P and the outer edge of the counter weight 38 is equal to the distance between the rotation axis P and the outer edge of the circumferential introduction line 33b or the distance between the rotation axis and the outer edge of the counter weight 38 is larger than the distance between the rotation axis and the outer edge of the circumferential introduction line 33b, the counter weight 38 corresponds to a protruding portion that forms a peripheral edge portion of the rotating unit.

Moreover, the rotating gantry 3 of this embodiment is formed in a thin shape so that the length L₁ of the rotating gantry of this embodiment in a longitudinal direction is smaller than the maximum outer diameter of the rotating unit (the diameter of a circulation track R₁, see Fig. 8). The length L₁ of the rotating gantry in the longitudinal direction is, for example, a distance L₁ between the front end of the first cylindrical portion 34 and the rear end of the second cylindrical portion 36. The maximum outer diameter of the rotating unit is a portion corresponding to the distance r₁ between the rotation axis P and the outer edge of the circumferential introduction line 33b (maximum outer diameter = radius rix2). Meanwhile, a portion corresponding to the distance between the rotation axis P and the outer edge of the counter weight 38 may be the maximum outer diameter.

The building 6 will be described below. As shown in Figs. 1 and 2, a cyclotron chamber 7 in which the cyclotron 2 is disposed, gantry chambers 8 in which the rotating gantries 3 are disposed, and a communication chamber 9 in which the guide line 4 is disposed are formed in the building 6. The building 6 is a building having, for example, a reinforced concrete structure or a steel skeleton concrete structure, and the respective chambers of the building are separated from each other by radiation shield walls made of concrete. The building 6 of this embodiment is formed in a rectangular shape in plan view. Meanwhile, in the respective drawings, the long-side direction of the building 6 is shown as an X direction, the short-side direction of the building 6 is shown as a Y direction, and the height direction of the building 6 is shown as a Z direction. Further, the upper side in Fig. 1 is described as the front side of the building 6.

For example, the cyclotron chamber 7 is disposed in one end portion of the building 6 in the long-side direction X. The cyclotron chamber 7 is formed in a rectangular shape in plan view, and is surrounded by a (radiation) shield wall 71. Front and rear walls of the cyclotron chamber 7 are disposed in the long-side direction X of the building 6, and side walls of the cyclotron chamber 7 are disposed in the short-side direction Y of the building 6. One side wall of the cyclotron chamber 7 serves as both the side wall of the building 6 and the rear wall of the cyclotron chamber 7.

Further, the cyclotron 2 is disposed on the front side of the cyclotron chamber 7, and a proton beam generated by the cyclotron 2 is directed from the rear side of the cyclotron 2. Furthermore, a communication chamber 9 is connected to the rear side of the cyclotron chamber 7.

The communication chamber 9 extends from the cyclotron chamber 7 in the long-side direction X of the building, 6. The communication chamber 9 is disposed adjacent to the rear sides of the plurality of gantry chambers 8. In this embodiment, the communication chamber 9 is disposed on the rearmost side of the building 6. Since the communication chamber 9 is partitioned by a radiation shield wall, the shield wall, which is positioned on the rear side of the communication chamber 9 extending in the long-side direction X, also serves as the rear wall of the building 6. Meanwhile, the shield wall, which is positioned on the front side of the communication chamber 9 extending in the long-side direction X, also serves as the rear wall of the gantry chamber 8. Further, the guide line 4, which extends in the communication chamber 9 in the long-side direction X, is branched at a predetermined position. The branched guide lines 4 extend so as to form a predetermined angle with respect to the long-slide direction X, and are led to the gantry chambers 8, respectively. A storage space for storing the guide line 4 may be formed at the communication chamber 9 along the branched guide lines 4.

The plurality of gantry chambers 8 is arranged in parallel in the long-side direction X of the building 6 so as to be adjacent to each other. The plurality of gantry chambers 8 is disposed on the front side of the communication chamber 9 so as to be adjacent to the communication chamber. Further, as shown in Fig. 1, the leftmost gantry chamber 8 is disposed adjacent to the cyclotron chamber 7, Furthermore, the length of the gantry chamber 8 in the long-side direction X is substantially the same as that of the adjacent gantry chamber 8 in the long-side direction X. Moreover, labyrinthine passages, which communicate with the gantry chambers 8, are formed on the front side of the gantry chambers 8.

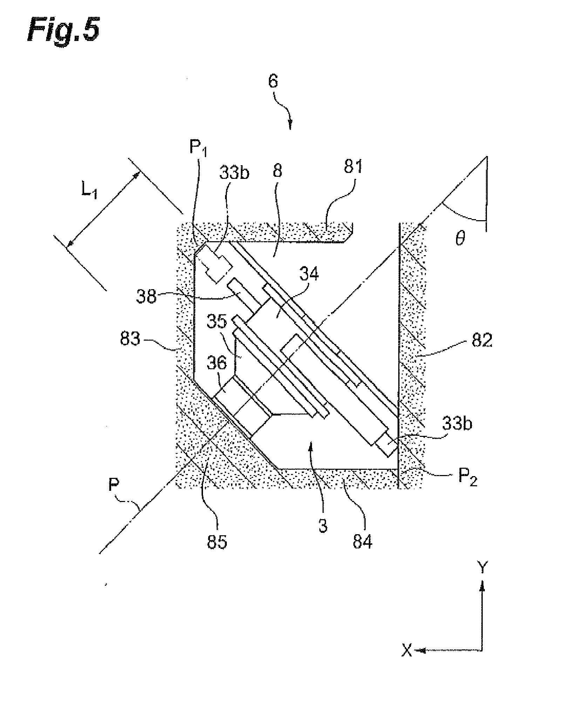

Fag. 5 is an enlarged plan view of the gantry chamber 8, As shown in Fig. 5, the gantry chamber 8 is formed in a substantially rectangular shape in plan view. For example, the gantry chamber 8 is formed in the shape of a pentagon that is obtained by cutting one corner portion of a tetragon. The gantry chamber 8 of this embodiment is formed in the shape of a pentagon that is obtained by cutting a left rear corner portion in Fig. 5. The gantry chamber 8 is partitioned by radiation shield walls.

The gantry chamber 8 includes a front wall 81, a right side wall 82, a left side wall 83, a first rear wall 84, and a second rear wall 85, as the radiation shield walls. The front wall 81 is disposed on the front side and extends in the long-side direction X. An inlet communicating with the inside of the gantry chamber 8 is formed at the front wall 81. The right and left side walls 82 and 83 are disposed so as to face each other, and extend in the short-side direction Y. The length of the right side wall 82 is different from that of the left side wall 83 in the short-side direction Y, and the right side wall 82 is longer than the left side wall 83. The right side wall 82 further extends to the rear side in the short-side direction Y as compared to the left side wall 83.

The first rear wall 84 is disposed on the rear side, extends in the long-side direction X, and faces the front wall 81. The first rear wall 84 is formed so as to extend from the rear end of the right side wall 82 and extend beyond the middle of the gantry chamber 8 in the long-side direction X.

The second rear wall 85 is disposed on the rear side, and extends in a direction that intersects the left side wall 83 and the first rear wall 84. The second rear wall 85 is formed so as to extend from the left end of the first rear wall 84 and extend to the rear end of the left side wall 83. The second rear wall 85 is disposed so as to be inclined with respect to the left side wall 83 and the first rear wall 84 at an angle of about 45°.

Further, in the above-mentioned gantry chamber 8, a diagonal line P₁P₂, which connects an intersection point P₁ between the front wall 81 and the left side wall 83 to an intersection point P₂ between the right side wall 82 and the first rear wall 84, corresponds to a portion of the gantry chamber 8 having the maximum width. In the gantry chamber 8, the diagonal line P₁P₂ intersects the long-side direction X and the short-side direction Y at an angle of about 45°. Further, in the gantry chamber 8 of this embodiment, the second rear wall 85 forms a surface parallel to the diagonal line P₁P₂.

Here, in the particle radiation therapy equipment 1 according to this embodiment, the portion of the rotating gantry 3 having the maximum width is disposed along the maximum width of an installation space of the rotating gantry 3. For example, the rotation track of a point, which is most distant from the rotation axis P of the rotating gantry 3, (the outer edge of the rotating unit of the rotating gantry 3) is disposed on a plane that is positioned on the diagonal line P₁P₂. Meanwhile, "on the diagonal line P₁P₂" includes not only a case where the rotation track is disposed in a diagonal direction in plan view but also a case where the rotation track is slightly deviated from the diagonal line P₁P₂.

The rotating gantry 3 of this embodiment is disposed so that the rotation axis P is inclined with respect to the long-side direction X and the short-side direction Y at a predetermined inclination angle θ. Specifically the rotation axis P of the rotating gantry 3 is inclined with respect to the long-side direction X at an inclination angle of about 45°.

Further, the rear side of the rotating gantry 3 is disposed so as to face the second rear wall 85, and the front side of the rotating gantry 3 faces the inlet of the gantry chamber 8. The inlet of the gantry chamber 8 is formed at a corner portion between the front wall 81 and the right side wall 82. Further, an area, which has a triangular shape in plan view, is formed on the front surface of the rotating gantry 3.

Furthermore, for example, the rotating gantry 3 is disposed so that inclined sides forming the outer edge of the cone portion 35 are parallel to the left side wall 83 and the first rear wall 84 in plan view as shown in Fig. 5.

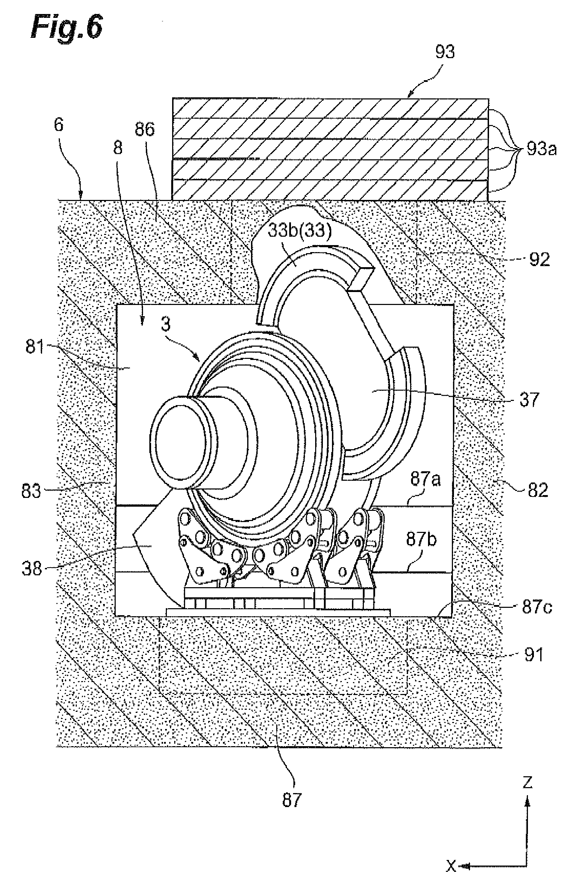

Figs. 6 to 8 are views showing the cross-section of the gantry chamber and the disposition of the rotating gantry in the gantry chamber. Further, as shown in Figs. 6 to 8, the gantry chamber 8 includes a ceiling 86 and a floor 87 as the radiation shield walls.

As shown in Fig. 7, a plurality of stepped portions is formed on the floor 87 of the gantry chamber 8. A first floor surface 87a that is formed on the front surface of the rotating gantry 3, a second floor surface 87b which is formed at a position lower than the first floor surface 87a and on which a support portion of the treatment table 31 is disposed, and a third floor surface 87c which is formed at a position lower than the second floor surface 87b and on which a support portion of the rotating gantry 3 is disposed are formed.

Here, cutout portions 91 and 92 are formed on the radiation shieldwalls of the gantry chamber 8 of this embodiment at positions corresponding to the circulation track R₂ of the counter weight 38 and/or the circulation track R₁ (see Fig. 8) of the introduction line 33 that are the rotating unit of the rotating gantry 3.

The cutout portion 91 is formed on the floor 87 at a position corresponding to the circulation track R₂ of the counter weight 38 and/or the circulation track R₁ of the introduction line 33 of the rotating gantry 3. The cutout portion 91 is a space that is recessed downward from the third floor surface 87c, and forms a movement space in which the counter weight 38 and/or the introduction line 33 of the rotating gantry 3 are moved. The cutout portion 91 is formed along the diagonal line P₁P₂ (the rotation direction of the protruding portion) in plan view.

The cutout portion 92 is formed on the ceiling 86 at a position corresponding to the circulation track R₂ of the counter weight 38 and/or the circulation track R₁ of the introduction line 33 of the rotating gantry 3. The cutout portion 92 is a space that is formed on the ceiling 86 and recessed upward, and forms a movement space in which the counter weight 38 and/or the introduction line 33 of the rotating gantry 3 are moved. The cutout portion 92 is formed along the diagonal line P₁P₂ (the rotation direction of the protruding portion) in plan view.

Further, the cutout portion 92 penetrates the ceiling 86 (the ceiling of the building 6) and is opened, and this opening is covered with a shield member 93, which is made of a separate material different from the material of the ceiling 86, from the outside of the gantry chamber 8 (the building 6). The shield member 93 is formed by stacking a plurality of shield plates 93a made of, for example, lead. Meanwhile, shield plates made of concrete may be stacked as the shield member 93, Furthermore, for example, a block body, which does not have the shape of a plate, may be used as the shield member.

Moreover, the shield, member 93 may be made of heavy concrete as a separate material. The shield member 93 made of heavy concrete is More expensive than the shield member 93 made of regular concrete, but has a high radiation shielding property. For example, when a shield member made of heavy concrete is used, the thickness of the shield member may be about 2/3 of the thickness of a shield member made of regular concrete, Further, if the shield member 93 modularized as a plate-like component is used, it may be possible to easily perform construction.

Further, since the cutout portion 92 is formed as an opening passing through the ceiling 86, the cutout portion may be used as a hatch through which the components of the rotating gantry 3 are carried.

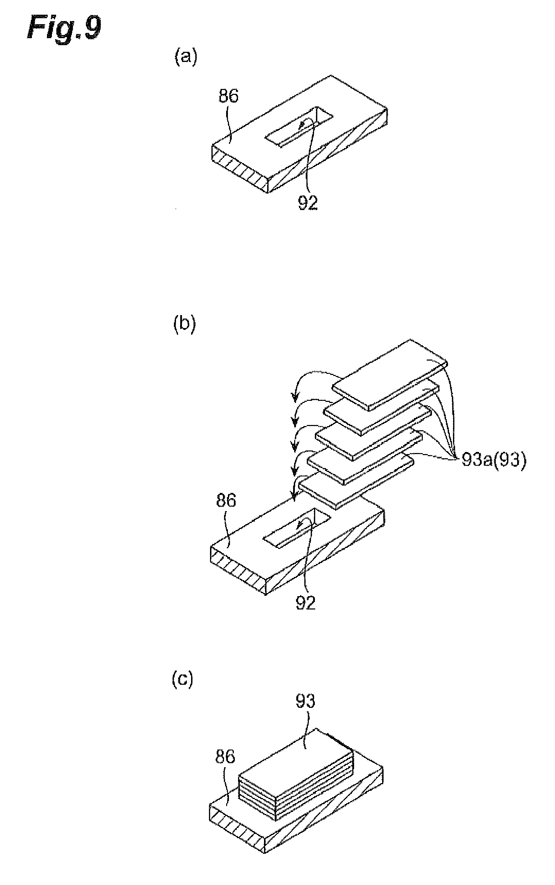

The procedure for constructing the shield member 93 will be described below with reference to Fig. 9. Fig. 9 shows only a part of the ceiling 86 where the cutout portion 92 is formed. As shown in Fig. 9A, an opening (cutout portion 92), which is formed at the ceiling 86 and is used to carry a gantry, is formed in a straight shape and is not provided with stepped portions. That is, the side walls of the opening are formed in a linear shape in a vertical direction.

Moreover, a plurality of shield plates 93a is superimposed on the cutout portion 92 as shown in Fig. 9B and, finally, the plurality of shield plates 93a is fixed to the outer surface of the ceiling 86 by using anchors or the like, so that the cutout portion 92 is shielded as shown in Fig. 9C.

Further, the cutout portion 92 passes through the ceiling in the vertical direction and stepped portions are not formed on the side walls of the cutout portion 92. Accordingly, when the components of the rotating gantry 3 are carried, damage to the components being carried, which is caused by bumping of the components against the stepped portion, is prevented.

Since the thickness of the portion of the thin rotating gantry 3, which has the maximum width, in the direction of the rotation axis, is small and the portion of the rotating gantry is disposed along the diagonal line P₁P₂ of the gantry chamber 8 in this embodiment, it may be possible to effectively utilize an installation space. Accordingly, since the length and width of the gantry chamber 8 may be reduced, the dimensions of the building 6 in the long-side direction X and the short-side direction Y may be reduced, so that the size of the building 6 is reduced, As a result, the construction costs of the building 6 are reduced. Moreover since it may be possible to effectively utilize an installation space, it may be possible to construct the particle radiation therapy equipment 1 at a site smaller than a site in the related art.

In the building 6 of this embodiment, the rotating gantry is disposed so that the rotation axis is inclined with respect to the long-side direction X and the short-side direction Y at an inclination angle of 45° in plan view. Accordingly, it may be possible to reduce the length of the equipment in the long-side direction X of the building 6 by 5 m for each rotating gantry 1. When three rotating gantries 3 are arranged in parallel in the long-side direction X, it may be possible to reduce the length of the equipment by 15 m.

Further, according to the particle radiation therapy equipment 1 of this embodiment, cutout portions are formed only at a part of the shield walls of the ceilings of the building 6 so as to correspond to the peripheral edge portions of the counter weights 38 and/car the introduction lines 33 that are the rotating units of the rotating gantries 3. Accordingly, when the rotating units of the rotating gantries 3 are rotated, the rotating units are moved in the cutout portions 91 and 92. Consequently, it may be possible to secure the movement spaces for the protruding portions that form the peripheral edge portions of the rotating gantries 3, and to obtain the gantry chamber 8 corresponding to the shapes of the rotating gantries 3. Therefore, it may be possible to reduce the dimensions of the gantry chamber 8 in the height direction of the gantry chamber. That is, it may be possible to lower the ceiling 86, and to facilitate the reduction of the size of the building 6 by removing the unnecessary space at upper portions of the gantry chambers 8. As a result, it may be possible to reduce the construction costs of the building 6.

Furthermore, in this embodiment, the rotating gantry 3 includes the circumferential introduction line 33b, which is curved in the circumferential direction and introduces accelerated particles into the irradiation unit 32, as a protruding portion, and the cutout portion 92 may store the circumferential introduction line 33b. Since the rotating gantry 3 includes the circumferential introduction line 33b that is curved to be twisted in the circumferential direction as described above, it may be possible to reduce the length of the protruding portion in the direction of the rotation axis and to reduce the width of the cutout portion 92 in the direction of the rotation axis.

In Figs. 1 and 2, the size of a building in the related art is shown as a comparison object by an imaginary line. In the past, for example, as for the dimensions of abuilding including three rotating gantries, the length of the building in the long-side direction X, that is, the width X₁ of the building was about 68 m; the length of the building in the short-side direction Y, that is, the depth Y₁ of the building was about 33 m; and the length of the building in the height direction Z, that is, the height Z₁ of the building was about 18 m. Meanwhile, as for the dimensions of the building 6 of this embodiment, the length of the building in the long-side direction X, that is, the width X₀ of the building was about 53 m; the length of the building in the short-side direction Y, that is, the depth Y₀ of the building was about 26 m; and the length of the building in the height direction Z, that is, the height Z₀ of the building was about 15 m. When the building 6 of this embodiment is compared with the building in the related art, it may be possible to reduce the volume of the building by about 50% and to significantly reduce the construction costs of the equipment.

Further, if this layout is employed, it may be possible to secure a triangular area of about 7 mx7 m in the gantry chamber 8, and to effectively utilize the area as a treatment space. Furthermore, an on-line PET system including a C-type arm, which protrudes from the ceiling portion to the rotating gantry 3, may be installed using this space.

A known on-line PET system is a technique for imaging the change of the shape of an affected part after treatment, and is a system that acquires a PET image by detecting a short-half-life positron nuclide emitted from the inside of the body of a patient immediately after the irradiation of a proton beam. Accordingly, it may be possible to accurately capture the change of the shape of a target tumor through the irradiation of a proton beam, and to prevent a normal tissue from being irradiated with a proton beam, As a result, it may be possible to further improve the accuracy of proton beam therapy.

If the rotating gantries 3 are obliquely disposed as described above, it may be possible to facilitate the effective use of a space and to improve the degree of freedom of the equipment.

The invention has been specifically described above with reference to the embodiment, but the invention is not limited to the above-mentioned embodiment. In the above-mentioned embodiment, the gantry chamber 8 has been formed in the shape of a pentagon, which is obtained by cutting one corner portion of a tetragon, in plan view, However, the gantry chamber 8 may be formed in other shapes. For example, the gantry chamber may be formed in a square shape, may be formed in other polygonal shapes such as a hexagonal shape, and corner portions of the gantry chamber may be rounded. Further, the shield walls facing each other may be disposed not to be parallel to each other.

Furthermore, the cutout portions 91 and 92 have been formed on a part of the radiation shield wall in the abode-mentioned embodiment, but the cutout portions 91 and 92 may be formed at the gantry chamber. In addition, the cutout portions may be formed on the side walls or the like.

Moreover, in the above-mentioned embodiment, the cutout portion 91 formed on the ceiling 86 has been formed so as to pass through the ceiling 86 in the height direction, However, the cutout portion 91 may not pass through the ceiling 86,

It should be understood that the invention is not limited to the above-described embodiment, but may be modified into various forms on the basis of the spirit of the invention. Additionally, the modifications are included in the scope of the invention.

## Claims

1. Accelerated particle irradiation equipment that performs irradiation of accelerated particles, the accelerated particle irradiation equipment comprising:
an irradiation device that includes a rotating unit rotatable about a rotation axis and performs irradiation of the accelerated particles generated by a particle accelerator; and
a building having an installation space in which the irradiation device is installed,
wherein the irradiation device is formed to be thin so as to have a small length in a direction of the rotation axis, and
a portion of the irradiation device, which has the maximum width in a radial direction orthogonal to the direction of the rotation axis, is disposed along the maximum width of the installation space.

2. The accelerated particle irradiation equipment according to claim 1,
wherein the irradiation device includes an irradiation unit that irradiates an irradiation target with the accelerated particles, an introduction line that introduces the accelerated particles into the irradiation unit, and a weight unit that is provided so as to face the introduction line with the rotation axis interposed therebetween and secures a weight balance between the weight unit and the introduction line, and
a distance between the rotation axis and a portion of the weight unit having the maximum outer diameter is smaller than a distance between the rotation axis and a portion of the introduction line having the maximum outer diameter.

3. The accelerated particle irradiation equipment according to claim 2,
wherein the rotating unit of the irradiation device includes a main body of the rotating unit that includes the irradiation unit, and a circumferential introduction line that is an introduction line curved in a circumferential direction on the outside of the main body of the rotating unit in the radial direction, and
the width of the weight unit in the direction of the rotation axis is smaller than that of the circumferential introduction line in the direction.of the rotation axis.

4. The accelerated particle irradiation equipment according to claim 1,
wherein the length of the irradiation device in the direction of the rotation axis is smaller than a radius of rotation of the irradiation device corresponding to the maximum width.
